(19) Europäisches Patentamt
European Patent Office
Office européen des brevets

(11) Veröffentlichungsnummer: **0 242 812**
**B1**

(12) # EUROPÄISCHE PATENTSCHRIFT

(45) Veröffentlichungstag der Patentschrift: **07.11.90**

(21) Anmeldenummer: **87105692.5**

(22) Anmeldetag: **16.04.87**

(51) Int. Cl.[5]: **A 61 K 47/10,** A 61 K 9/66, A 61 K 31/685

(54) **Verwendung von Glycofurol zum Verflüssigen von Arzneimittelzubereitungen für das Abfüllen in Weichgelatinekapseln.**

(30) Priorität: **24.04.86 DE 3613799**

(43) Veröffentlichungstag der Anmeldung:
**28.10.87 Patentblatt 87/44**

(45) Bekanntmachung des Hinweises auf die Patenterteilung:
**07.11.90 Patentblatt 90/45**

(84) Benannte Vertragsstaaten:
**AT BE CH DE ES FR GB GR IT LI LU NL SE**

(56) Entgegenhaltungen:
**EP-A-0 143 857**
**EP-A-0 147 741**
**DE-A-2 949 849**

(73) Patentinhaber: **A. Nattermann & Cie. GmbH**
**Nattermannallee 1**
**D-5000 Köln 30 (DE)**

(72) Erfinder: **Dürr, Manfred, Dr.**
**Am blauen Stein 10**
**D-5024 Pulheim/Dansweiler (DE)**
Erfinder: **Fribolin, Hans Ulrich**
**Im Jagdfeld 45**
**D-4040 Neuss (DE)**
Erfinder: **Harhausen, Ekkehard, Dr.**
**Am Dörenkamp 1**
**D-2402 Sarkwitz (DE)**
Erfinder: **Seidel, Jürgen, Dr.**
**Venloer Strasse 67**
**D-5023 Pulheim (DE)**

(74) Vertreter: **Sternagel, Hans-Günther, Dr. et al**
**Patentanwälte Dr. Michael Hann Dr. H.-G. Sternagel Sander Aue 30**
**D-5060 Bergisch Gladbach 2 (DE)**

**EP 0 242 812 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Gegenstand der vorliegenden Erfindung ist die Verwendung von Glycofurol zur Verflüssigung von mehr als 50% Phosphatidylcholin mit einem hohen Gehalt an ungesättigten Fettsäuren enthaltenden Arzneimittelzubereitungen, die neben Phosphatidylcholin einen weiteren aktiven Wirkstoff enthalten können, wobei die dabei erreichte Viskosität das Abfüllen in Weichgelatinekapseln ermöglicht. Phosphatidylcholin ist neben anderen Phospholipiden Bestandteil der handelsüblicen Lecithine und besitzt aufgrund seiner therapeutischen und ernährungsphysiologischen Anwendung besondere Bedeutung.

Die handelsüblichen Lecithin-Präparate enthalten 35 bis maximal 55 Gew.% Phospholipide neben pflanzlichen Ölen und anderen üblichen Hilfsstoffen, wobei die Phospholipide natürliche Gemische verschiedener Phospholipide, wie Phosphatidylcholin, Phosphatidylethanolamin, Phosphatidylinosit usw., darstellen. Die Präparate werden als flüssige Zubereitungen oder Granulate eingenommen. Für die therapeutisch Anwendung sind jedoch Präparate wünschenswert, die einen hohen Anteil an reinem Phosphatidylcholin enthalten, da sich insbesondere Phosphatidylcholin bei der Behandlung von Fettstoffwechselstörungen, Lebererkrankungen und zerebralen Erkrankungen bewährt hat. Besondere Bedeutung haben Phosphatidylcholine mit einem hohen Gehalt an ungesättigten und essentiellen Fettsäuren, welche durch die folgende allgemeine Formel veranschaulicht werden können:

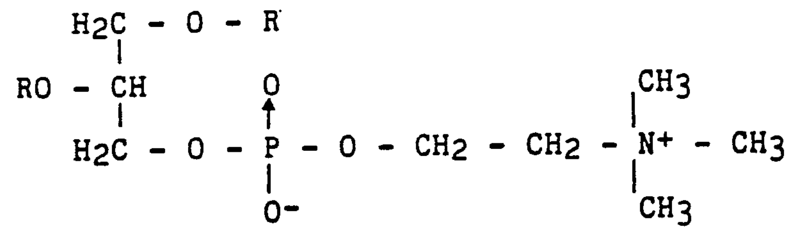

wobei R = Acylreste natürlicher Fettsäuren, wie Palmitinsäure, Stearinsäure, Ölsäure, Linolsäure, Linolensäure und Arachidonsäure, darstellen, wobei der Gehalt an ungesättigten Fettsäuren über 70% beträgt. Nun sind diese Phosphatidylcholine aber plastische Massen, die technisch schwere zu verarbeiten und schlecht zu handhaben sind und deshalb in dieser Form einen erheblichen Nachteil besitzen. Zur therapeutischen Anwendung muß das Phosphatidylcholin in eine Form gebracht werden, die eine galenische Zubereitung ermöglicht.

Bei der Wahl der Darreichungsform spielt allgemein die biologische Verfügbarkeit eine große Rolle, wobei in der Regel Lösungen eines Wirkstoffes biologisch besser verfügbar sind. Schwierigkeiten bei den Herstellung von Lösungen treten häufig dann auf, wenn der Wirkstoff wasserunlöslich ist. Bei der Auswahl eines geeigneten Lösungsmittels spielt neben dem ausreichenden Lösevermögen insbesondere die physiologische Verträglichkeit eine entscheidende Rolle.

Zur Überführung von Phosphatidylcholin in eine orale Darreichgungsform wird ein Lösungsmittel benötigt, das — neben gutem Lösevermögen und physiologischer Unbedenklichkeit — technisch gut zu verarbeitende Lösungen liefert. Die Lösungen dürfen eine bestimmte Viskosität nicht überschreiten, um ein problemloses Abfüllen, z.B. in Weichgelatinekapseln, zu ermöglichen.

Das Problem der Verflüssigung von Phosphatiden ist in der Vergangenheit schon mehrfach angegangen worden. So wird in DE—PS 286 061 ein Verfahren zur Herstellung hochprozentiger Lösungen von Lecithin in Ölen und Fetten beschrieben. Als Lösungsmittel dient eine Mischung aus Ölsäure und Olivenöl. Man erhält eine 40%ige Lecithinlösung. Bei einem anderen Verfahren (DE—AS 12 27 191) zur Herstellung von flüssigen Lecithinpräparaten werden Polyalkohole (Mannit, Arabit, Sorbit) in Verbindung mit Ethanol benutzt. Die erhaltenen Lösungen besitzen aber nur einen Lecithingehalt von maximal 10%. Das US—Patent 2 555 972 beschreibt eine Phosphatidmischung niedriger Viskosität, bestehend aus 50 bis 60% Phosphatiden, 2 bis 4% höheren Fettsäuren, 30 bis 47% pflanzlichen Ölen und 1 bis 6% Propylenglycol. Phosphatide können nach US—Patent 2 483 748 auch mit Fettsäureestern, wie Sojaölfettsäuremethylester, Ölsäureester u.a. verflüssigt werden. Man erhält Phosphatidlösungen mit bis zu 80% Phosphatidgehalt, die bei Raumtemperatur fließfähig sind. Alle diese Verfahren eignen sich mehr oder weniger gut zur Verflüssigung von handelsüblichen lecithinen. Nun ist handelsübliches Lecithin aber eine Phosphatidmischung, bestehend aus Öl, Phosphatidylcholin, Phosphatidylethanolamin, Phosphatidylinosit und anderen Begleitlipiden.

Die obenerwähnten Verfahren gehen entweder von diesem Rohlecithin oder von entöltem Rohlecithin aus. In jedem Fall handelt es sich bei den bekannten Verfahren um die Verflüssigung einer Rohphosphatidmischung. Rohphosphatidmischungen haben aber ein anderes Löseverhalten als deren reine Komponenten. Die in den bekannten Verfahren zur Herstellung von Lecithinlösungen angegebenen Lösungsmittel lassen sich deshalb nicht in gleicher Weise auf reines Phosphatidylcholin übertragen.

In DE—PS 1141 639 wird Phosphatidylcholin durch Komplexieren mit Gallensäuren wasserlöslich gemacht. Nachteil ist jedoch, daß das Verhältnis von Phosphatidylcholin zu Gallensäure 1:1 beträgt. Auch in DE—OS 2556 592 werden flüssige Zubereitungen aus Phosphatidylcholin, Gallensäuren, Sonnenblumenöl und Monoglyceriden, die in Weichgelatinekapseln abgefüllt werden können, beschrieben.

Hierbei werden jedoch nur Phosphatidylcholingehalte von weniger als 20 Gew.% erreicht.

In DE—OS 2949849 werden hochkonzentrierte Phosphatidylcholinlösungen beschrieben, bei denen als Verflüssiger Milchsäureethylester eingesetzt wurden. Milchsäureethylester ist jedoch in Gegenwart geringer Mengen Wasser sehr instabil, so daß es in der Weichgelatinekapsel zur Spaltung in Milchsäure und Ethylalkohol kommt, die Milchsäure führt durch Erniedrigung des pH-Wertes zur Abspaltung der Fettsäure im Phosphatidylcholin und es kommt zur Bildung von unerwünschtem Lysophosphatidylcholin. Der gleichzeitig anstehende Ethylalkohol führt zusätzlich zur Versprödung der Kapselhülle.

Da sich die Herstellung geeigneter Phosphatidylcholin-Füllmassen für Weichgelatinekapseln so außerordentlich schwierig gestaltet hat man versucht, anstelle von Weichgelatinekapseln Hartgelatinekapseln einzusetzen. In DE—OS 30 22 136 sind phosphatidylcholinhaltige Abfüllmassen beschrieben, die durch Zugabe von Triglyceriden und Ethylalkohol verflüssigt werden, wobei das Ethanol beim Abfüllen verdampft. Diese Massen sind jedoch nicht geeignet für die Abfüllung in Weichgelatinekapseln. Für die therapeutische Anwendung haben sich jedoch Weichgelatinekapseln aufgrund ihrer guten Handhabbarkeit, ihrem schnellen Auflösevermögen nach der Einnahme sowie ihrem Vermögen, für die abgefüllten Wirkstoffe besseren Schutz insbesondere gegen Oxydation bei längerem Lagern insbesondere in höheren Klimazonen zu bieten, bewährt.

Aus EP—A—143 857 ist es bekannt Niefedipin in Glycofurol aufzulösen, um es als flüssige Kapselfüllung für Weichgelatinekapseln einzusetzen.

Aufgabe der vorliegenden Erfindung war es deshalb, phosphatidylcholinhaltige Füllmassen für Weichgelatinekapseln zu entwickeln, die mehr als 50 Gew.% Phosphatidylcholin enthalten und flüssige Stoffe enthält, die sowohl mit dem Wirkstoff als auch mit der Kapsel verträglich sind.

Diese Aufgabe wird durch Verwendung von flüssigen Arzneimittelzusammensetzungen, die in Weichgelatinekapseln eingeführt werden, wobei die Arzneimittelzubereitungen in Gew.% bezogen auf Gewicht der Gesamtzusammensetzung enthalten:

| | |
|---|---|
| 50—94% | Phosphatidylcholin, dessen Acylrest aus natürlichen Fettsäuren, wie Palmitinsäure, Stearinsäure, Ölsäure, Linolsäure, Linolensäure und Arachidonsäure bestehen, wobei der Gehalt an ungesättigten Fettsäuren über 70% beträgt, |
| 20—2 Gew.% | Glycofurol und |
| 30—4 Gew.% | pflanzliche Öle und/oder ein oder mehrere Alkanole als weitere Verflüssiger, Antioxydantien, Aromastoffe und weitere pharmazeutische Wirkstoffe. |

Es wurde nun überraschend gefunden, daß man phosphatidylcholinhaltige Füllmassen mit hohem Phosphatidylcholingehalt für Weichgelatinekapseln erhalten kann, wenn man als Verflüssiger Glycofurol einsetzt. Glycofurol (Tetrahydrofurylalkoholpolyethylenglykolether)

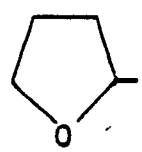 $CH_2(O-CH_2-CH_2)_nOH$ n = 2

ist ein bekanntes Lösungsmittel, ist toxikologisch unbedenklich und besitzt keine eigene pharmakodynamische Wirksamkeit.

Bei der Vielzahl der bisher eingesetzten Lösevermittler sowie der zur Verfügung stehenden Lösungsvermittler muß es als überraschend angesehen werden, daß gerade Glycofurol allen Anforderungen zum Abfüllen von Phosphatidylcholin dessen Acylreste aus natürlichen Fettsäuren bestehen, wobei der Gehalt an ungesättigten Fettsäuren über 70% beträgt, in Weichgelatinekapseln genügt.

Als Phosphatidylcholine werden natürliche, aus z.B. Sojabohnen oder Ei gewonnene Phosphatidylcholine mit hohem Gahalt und ungesättigten essentiellen Fettsäuren eingesetzt. Es können jedoch auch andere Phosphatidylcholine natürlicher oder synthetischer Herkunft eingesetzt werden wenn der gehelt aus ungesaltigten Fetsäuren in den Acylresten uber 70% beträgt.

Der Phosphatidylcholingehalt der Füllmasse beträgt 50—94 Gew.%, bevorzugt 70 bis 79 Gew.%. Zur Verflüssigung werden 2—20 Gew.%, bevorzugt 5—20 Gew.% glykofurol, besonders bevorzugt 16—18 Gew.%, zugesetzt. Zur besseren Verarbeitung werden noch bis zu 12 Gew.% natürliche Öle, insbesondere Sojaöl, bis zu 10 Gew.% 1,2-Propandiol und 0,3—2 Gew.% Ethanol und weitere übliche Hilfsstoffe wie Antioxidantien, Aromastoffe zugesetzt.

Es ist auch möglich, weitere Wirkstoffe mitzuverarbeiten.

Eine besonders bevorzugte Füllmasse enthält 70 bis 79 Gew.% Phosphatidylcholin, 16 bis 18 Gew.% glycofurol, 4 bis 6 Gew.% Sojaöl, 4 bis 5,5 Gew.% 1,2-Propandiol und 0,5 bis 1 Gew.% Ethanol.

Nach einer Ausführungsform der erfindungsgemäßen Verwendung ist die Füllsmasse mit 0,1 bis 10% einer physiologisch aktiven Substanz versetzt.

Als physiologisch aktive Substanzen, die dem Phosphatidylcholin zugemischt werden können, kommen z.B. in Frage:

Pflanzenextrakte, wie z.B. Chelidonium, Crataegus, Curcuma, Kastanien, Betula, Beerentrauben- oder Sennaextrakte;

fett- oder wasserlösliche Vitamine, wie z.B. Vitamin C, Vitamin A, E, F, B-Komplexe bzw. $B_{12}$;

ätherische Öle, wie z.B. aus Fenchel, Kümmel, Pfefferminz, Eukalyptus;

Purine, wie z.B. Theophyllin, Theobromin oder Coffein;

Alkaloide, wie z.B. Chinidin, Ephedrin, Codein, Atropin, Papaverin, Morphine, Reserpin, Strychnin, Mutterkornalkaloide, besonders Ergotamin, Ergocristin, Ergocornin, Raubasin, Dihydroergotoxin, Dihydroergotamin oder Dihydroergocristin;

Gallensäuren, wie z.B. Desoxycholsäure oder Taurocholsäure;

Saponine, wie z.B. Aescin;

Hormone, wie z.B. Methyltestosteron oder Ethinylöstrandiol;

Sulfonamide oder Antibiotika, wie z.B. Penicillin oder Cephalosporine;

Glykoside, wie z.B. Herzglykoside, besonders Digoxin, Digitoxin oder Strophantin;

nichtsteroidale Antirheumatika, wie z.B. Acetylsalicylsäure, Indometacin, Diclofenac oder Piroxicam;

oder andere pharmazeutisch relevanten Stoffe.

Zur Herstellung der neuen Füllmasse wird das Phosphatidylcholin nach überlichen Verfahren zerkleinert und in einem Gemisch aus Alkandiol, wie z.B. Propandiol oder Butandiol, und Glycofurol und ggf. einem natürlichen Öl und Erwärmen bis 70°C durch Rühren in Lösung gebracht. Anschließend wird ggf. zur Einstellung der benötigten Viskosität noch Ethanol zugesetzt. Das Phosphatidylcholin kann auch in ethanol gelöst werden und die sonstigen Lösungsvermittler und Hilfsstoffe zugegeben werden. Anschließend wird das überschüssige Ethanol abgezogen. In dieses Füllgut werden die Wirksubstanzen in einem geeigneten Mischer homogen eingearbeitet.

Die Abfüllung der so erhaltenen Füllmassen in Weichgelatinekapseln erfolgt nach an sich bekannten Verfahren (s. J. P. Stanley 'Soft Gelatin Capsules' in 'The theory and practice of indus. Pharm.' Herausgeber L. Lachman, Verlag LEA & Febiger Philadelphia 1976) wie z.B. dem Rotary Die-Verfahren und den allgemein bekannten Abfüllbedingungen.

## Beispiel 1

Herstellung von 5,36 kg Füllmasse

| | |
|---|---|
| Phosphatidylcholin | 72 Gew.-% |
| Glycofurol | 17 Gew.-% |
| Propandiol-1,2 | 5 Gew.-% |
| Sojaöl | 5,5 Gew.-% |
| Ethanol | 0,5 Gew.-% |

Das zähplastische Phosphatidylcholin wird zerkleinert und in dem Gemisch aus Glycofurol, Propandiol-1,2 und Sojaöl unter Erwärmen bis 70°C durch Rühren in Lösung gebracht. Anschließend wird das Ethanol zugesetzt.

Die so erhaltene Füllmasse wird nach dem Rotary Die-Verfahren in 8000 Weichgelatinekapseln der Größe 11 minims oblong abgefüllt. Füllgewicht pro Kapsel 670 mg.

## Beispiel 2

| | |
|---|---|
| Phosphatidylcholin | 79 Gew.-% |
| Glycofurol | 17 Gew.-% |
| Sojaöl | 3 Gew.-% |
| Ethanol | 1 Gew.-% |

Das Phosphatidylcholin wird in einem Überschuß von Ethanol gelöst. Anschließend werden das Glycofurol und das Sojaöl eingerührt und das überschüssige Ethanol abgedampft bis auf einem Gehalt von 1 Gew.-% in der Gesamtmasse.

Die erhaltene Füllmasse wird nach dem Rotary Die-Verfahren in Weichgelatinekapseln der Größe 8 minims oblong abgefüllt.

Füllgewicht pro Kapsel 500 mg.

Beispiel 3

| | |
|---|---|
| Phosphatidylcholin | 67 Gew.-% |
| Glycofurol | 15 Gew.-% |
| Sojaöl | 12 Gew.-% |
| Propandiol-1,2 | 5,7 Gew.-% |
| Ethanol | 0,3 Gew.-% |

Die Herstellung der Füllmassen sowie das Abfüllen in Weichgelatinekapseln erfolgt gemäß Beispiel 1.

Beispiel 4

| | |
|---|---|
| Phosphatidylcholin | 72,5 Gew.-% |
| Glycofurol | 20 Gew.-% |
| Sojaöl | 6,5 Gew.-% |
| Ethanol | 1 Gew.-% |

Die Herstellung der Füllmassen sowie das Abfüllen in Weichgelatinekapseln erfolgt gemäß Beispiel 2.

Beispiel 5

| | |
|---|---|
| Phosphatidylcholin | 72,5 Gew.-% |
| Glycofurol | 9,5 Gew.-% |
| Sojaöl | 11,5 Gew.-% |
| Propandiol-1,2 | 5 Gew.-% |
| Ethanol | 1,5 Gew.-% |

Die Herstellung der Füllmassen sowie das Abfüllen in Weichgelatinekapseln erfolgt gemäß Beispiel 2.

Beispiel 6

Phosphatidylcholin + Wirkstoff

| | |
|---|---|
| Zusammensetzung: | 3 % Piroxicam |
| | 70 % Phosphatidylcholin |
| | 16 % Glycofurol |
| | 5 % Propandiol |
| | 5,5% Sojaöl |
| | 0,5% Ethanol |

Herstellung:

In das nach Beispiel 1 hergestellte Füllgut wird Piroxicam in einem geeigneten Mischer homogen eingearbeitet. Es wurden Weichgelatinekapseln der Größe 11 minims oblong hergestellt mit einem Füllgewicht von 670 mg.

Beispiel 7

Phosphatidylcholin + Wirkstoff

| | |
|---|---|
| Zusammensetzung: | 5,0% Diclofenac-Natrium |
| | 68,5% Phosphatidylcholin |
| | 15,5% Glycofurol |
| | 5,0% Propandiol |
| | 5,5% Sojaöl |
| | 0,5% Ethanol |

Herstellung:

In das nach Beispiel 2 hergestellte Füllgut wird das Diclofenac-Natrium in einem geeigneten Mischer homogen eingearbeitet. Es wurden Weichgelatinekapseln der Größe 9 minims oblong hergestellt mit einem Füllgewicht von 500 mg.

Beispiel 8

Phosphatidylcholin + Wirkstoff

| | |
|---|---|
| Zusammensetzung: | 7% Ethophyllin |
| | 70% Phosphatidylcholin |
| | 17% Glycofurol |
| | 2% Propandiol |
| | 3% Sojaöl |
| | 1% Ethanol |

Herstellung:

In das nach Beispiel 1 hergestellte Füllgut wird das Ethophyllin homogen eingearbeitet. Es wurden Weichgelatinekapseln der Größe 11 minims oblong hergestellt mit einem Füllgewicht von 715 mg.

Beispiel 9

Phosphatidylcholin + Wirkstoff

| | |
|---|---|
| Zusammensetzung: | 0,5% Dihydroergotaminmethansulfonat |
| | 71,0% Phosphatidylcholin |
| | 11,0% Glycofurol |
| | 11,0% Propandiol |
| | 5,0% Sojaöl |
| | 1,5% Ethanol |

Herstellung:

Phosphatidylcholin wird in einem Überschuß von Ethanol gelöst, anschließend wird eine Lösung von dihydroergotaminmethansulfonat in Glycofurol und Propandiol zugesetzt. Nach Zugabe des Sojaöls wird das Ethanol bis auf den Sollwert abdestilliert. Es wurden Weichgelatinekapseln der Größe 4 minims oval hergestellt mit einem Füllgewicht von 200 mg.

Beispiel 10

Phosphatidylcholin + Wirkstoff

| | |
|---|---|
| Zusammensetzung: | 0,1% Digoxin |
| | 77,0% Phosphatidylcholin |
| | 15,0% Glycofurol |
| | 3,0% Propandiol |
| | 4,0% Sojaöl |
| | 0,9% Ethanol |

Herstellung:

Entsprechend Beispeil 9. Es wurden Weichgelatinekapseln der Größe 4 minims oblong hergestellt mit einem Füllgewicht von 200 mg.

**Patentansprüche**

1. Verwendung von Glycofurol bei der Herstellung von flüssigen Arzneimittelzusammensetzungen, die in Weichgelatinekapseln eingeführt werden, wobei die Arzneimittelzubereitungen in Gew.% bezogen auf Gewicht der Gesamtzusammensetzung enthalten:

| | |
|---|---|
| 50—94% | Phosphatidylcholin, dessen Acylrest aus natürlichen Fettsäuren, wie Palmitinsäure, Stearinsäure, Ölsäure, Linolsäure, Linolensäure und Arachidonsäure bestehen, wobei der Gehalt an ungesättigten Fettsäuren über 70% beträgt, |
| 20—2 Gew.% | Glycofurol und |
| 30—4 Gew.% | pflanzliche Öle und/oder ein oder mehrere Alkanole als weitere Verflüssiger, Antioxydantien, Aromastoffe und weitere pharmazeutische Wirkstoffe. |

2. Verwendung nach Anspruch 1, dadurch gekennzeichnet, daß die Füllmasse 50 bis 94 Gew.% Phosphatidylcholin, 2 bis 20 Gew.% Glycofurol, 0 bis 12 Gew.% Sojaöl, 0 bis 10 Gew.% 1,2-Propandiol und 0,3 bis 2 Gew.% Ethanol enthält.

3. Verwendung nach Anspruch 1, dadurch gekennzeichnet, daß die Füllmasse 70 bis 79 Gew.% Phosphatidylcholin, 16 bis 18 Gew.% Glycofurol, 4 bis 6 Gew.% Sojaöl, 4 bis 5,5 Gew.% 1,2-Propandiol und 0,5 bis 1 Gew.% Ethanol enthält.

4. Verwendung nach Ansprüchen 1 bis 3, dadurch gekennzeichnet, daß die Füllmasse mit 0,1 bis 10% einer physiologisch aktiven Substanz versetzt ist.

**Revendications**

1. Utilisaiton du glycofurol dans la réalisation de préparations pharmaceutiques liquides qui sont introduites dans des capsules de gélatine molle, les préparations pharmaceutiques contenant en % par rapport au poids de la préparation totale:

| | |
|---|---|
| 50—94% | de phosphatidylcholine dont les radicaux acyle sont constitués d'acides gras naturels, comme l'acide palmitique, l'acide stéarique, l'acide oléique, l'acide linoléique, l'acide linolénique, et l'acide arachidique, la teneur en acides gras insaturés dépassant 70%. |
| 20—2% | en poids de glycofurol, et |
| 30—4% | en poids d'huiles végétales et/ou un ou plusieurs alcools, comme autres agents de liquéfaction, des agents antioxydants, des produits aromatiques et autres agents pharmaceutiques. |

2. Utilisation selon la revendication 1, caractérisée par le fait que la masse de remplissage contient 50 à 94% en poids de phosphatidylcholine, 2 à 20% en poids de glycofurol, 0 à 12% en poids d'huile de soja, 0 à 10% en poids de 1,2-propanediol et 0,3 à 2% en poids d'éthanol.

3. Utilisation selon la revendication 1, caractérisée par le fait que la masse de remplissage contient 70 à 79% en poids de phosphatidylcholine, 16 à 18% en poids de glycofurol, 4 à 6% en poids de huile de soja, 4 à 5,5% en poids de 1,2 propanediol et 0,5 à 1% d'éthanol.

4. Utilisation selon les revendications 1 à 3, caractérisée par le fait que l'on mélange la masse de remplissage avec 0,1 à 10% en poids d'une substance physiologiquement active.

**Claims**

1. The use of glycofurol in preparing liquid pharmaceutical compositions, which are filled into soft gelatin capsules, where the weight % of the pharmaceutical preparations based on the total weight of the composition comprises:

| | |
|---|---|
| 50—94 weight % | phosphatidyl choline, the acyl radical of which consists of natural fatty acids, such as, palmitic acid, stearic acid, oleic acid, linoleic acid, linolenic acid and arachidonic acid, and the content of unsaturated acids being more than 70%, |
| 29— 2 weight % | glycofurol and |
| 30— 4 weight % | vegetable oils and/or one or more alkanols as further liquefying agents, antioxidants, flavourings and other pharmaceutically active ingredients. |

2. The use according to claim 1, characterized in that the filling mass comprises 50 to 94 weight % phosphatidyl choline, 2 to 20 weight % glycofurol, 0 to 12 weight % soya oil, 0 to 10 weight % 1.2-propandiol and 0.3 to 12 weight % ethanol.

3. The use according to claim 1, characterized in that the filling mass comprises 70 to 79 weight % phosphatidyl choline, 16 to 18 weight % glycofurol, 4 to 6 weight % soya oil, 4 to 5.5 weight % 1.2-propandiol and 0.5 to 1 weight % ethanol.

4. The use according to claims 1 to 3, characterized in that the filling mass is charged with 0.1 to 10% of a physiologically active substance.